# EUROPÄISCHE PATENTSCHRIFT

(11) **EP 1 555 959 B1**
(45) Veröffentlichungstag und Bekanntmachung des Hinweises auf die Patenterteilung: **05.03.2008**
(21) Anmeldenummer: 03769192.0
(22) Anmeldetag: 16.09.2003
(51) Int. Cl.: A61F 2/90

(54) **STENT ZUR IMPLANTATION IN ODER UM EIN HOHLORGAN**
STENT TO BE IMPLANTED WITHIN OR AROUND A HOLLOW ORGAN
ENDOPROTHESE A IMPLANTER DANS OU AUTOUR D'UN ORGANE CREUX

(30) Priorität: 17.09.2002 DE 10243136
(43) Veröffentlichungstag der Anmeldung: 27.07.2005
(73) Patentinhaber: Light-Cut GmbH & Co. KG, 66620 Nonnweiler-Otzenhausen (DE)
(72) Erfinder: JUNG, Johannes, 76229 Karlsruhe (DE)
(74) Vertreter: Fritzsche, Thomas
(86) Internationale Anmeldenummer: PCT/DE2003/003067
(87) Internationale Veröffentlichungsnummer: WO 2004/026176

(56) Entgegenhaltungen:
- EP-A- 0 928 605
- WO-A-00/47134
- WO-A-01/89414
- WO-A-02/13725
- WO-A-99/65418
- US-A- 5 707 386
- US-A- 5 948 016

## Beschreibung

Die Erfindung betrifft einen Stent zur Implantation in oder um ein Hohlorgan, insbesondere einen selbstexpandierenden Stent, mit mehreren ringförmigen Wandsegmenten, die eine elastische Struktur aufweisen und die mittels Verbindungselementen miteinander verbunden sind.

Ein Stent dieser Art wird beispielsweise in der DE 197 46 882 A1 beschrieben. Stents werden in ein Hohlorgan, wie z. B. Blutgefäße, Harnleiter, Speiseröhren oder Gallenwege eingesetzt, um zu gewährleisten, dass diese Hohlorgane eine lichte Weite beibehalten. Insbesondere bei Blutgefäßen kommt es häufig durch Ablagerungen zu einem Verschließen des Blutgefäßes. Der Blutdurchfluss wird dadurch verhindert, was schwerwiegende Folgen hat. In ein solches erkranktes Hohlorgan wird dann mittels eines Katheters ein solcher Stent eingesetzt. Häufig werden beispielsweise auch selbstexpandierende Stents verwendet, die in einem ersten Zustand mit geringem Durchmesser in das Hohlorgan eingeführt und in dieser Position dann in einen zweiten Zustand mit größerem Durchmesser expandieren oder expandiert werden. Stents kommen aber auch dann zum Einsatz, wenn ein Hohlorgan, wie beispielsweise ein Blutgefäß, nicht mehr genügend Festigkeit aufweist, um in seiner ursprünglichen Form zu verbleiben. Dies führt zu sogenannten Aneurismen. In diesem Fall besteht das Risiko, dass sich das Hohlorgan, nämlich das Blutgefäß, an einer derartig erkrankten Stelle aufweitet und dabei reißen kann. Dies führt zu einer unerwünschten inneren Blutung. Um eine solche innere Verletzung zu vermeiden, wird ein Stent, insbesondere ein beschichteter Stent, derart in das erkrankte Gefäß eingesetzt, dass das erweiterte aneurysmatisch veränderte Segment vom Stent überbrückt wird. Die beiden Enden des Stents dichten dann jeweils das davor und dahinter liegende gesunde Segment ab.

Ein solcher Stent muss einerseits genügend Flexibilität aufweisen, um Bewegungen seines Trägers oder der Blutgefäße bzw. Hohlorgane folgen zu können. Gerade bei selbstexpandierenden Stents ist es auch erforderlich, dass der Stent auf den geringen Durchmesser in dem ersten Zustand komprimiert wird und dass er dann auf den großen Durchmesser bei dem zweiten Zustand expandieren kann. Diese Flexibilität wird dadurch gewährleistet, dass mehrere elastische, ringförmige Wandsegmente mittels einzelner Verbindungselemente miteinander verbunden sind. Nachteilig bei dem aus der DE 19746882 A1 bekannten Stent ist es, dass eine Kontraktion, beispielsweise bei seiner Plazierung im Gefäß, eine Längenänderung des Stents zur Folge hat. Dies kann z. B. dann auftreten, wenn der Stent unter Zugspannung eingesetzt wird und sich dann verkürzt, wobei es vorkommen kann, dass das verengte oder erweiterte Segment nicht mehr vom Stent überdeckt wird. Eine weitere Gefahr besteht dadurch, dass ein Stentende eines unter Zugspannung stehenden Stents aus dem behandelten Gefäß herausragt, wenn es sich zum Beispiel um eine Veränderung an einem Gefäßabgang handelt.

Ziel der Erfindung ist es deshalb, einen Stent anzugeben, der flexibel und nachgiebig ist, bei dem aber auf eine Zugspannung oder Druckspannung oder in Folge einer Kontraktion möglichst nur eine geringe oder keine Längenänderung auftritt.

Das Ziel wird nach der in Anspruch 1 definierten Erfindung dadurch erreicht, dass bei einem Stent der eingangs genannten Art die Verbindungselemente mindestens einen durchgehenden Längssteg bilden, der mindestens eine Komponente in Axialrichtung zum Aufnehmen einer Druckspannung oder einer Zugspannung in Längsrichtung hat, so dass seine Länge im wesentlichen konstant bleibt.

Auf diese Weise besteht der Stent einerseits aus den elastischen ringförmigen Wandsegmenten, wodurch er den Bewegungen des Hohlorgans folgen kann, in das er implantiert ist. Andererseits sind die einzelnen Wandsegmente mittels eines durchgehenden Längsstegs miteinander verbunden, der Druckspannungen oder Zugspannungen in Längsrichtung aufnehmen kann, ohne dass dadurch eine Längenänderung des Stents bewirkt wird. Gleichermaßen bewirkt ein Kontrahieren, also ein Zusammendrücken der einzelnen Wandsegmente ebenfalls keine Längenänderung des Stents, da die dabei eventuell auftretenden Zugspannungen oder Druckspannungen auf den durchgehenden Längssteg übertragen und von diesem aufgenommen werden.

Ein solcher Stent ist aus der US-A-5707386 bekannt.

Eine Weiterbildung der Erfindung zeichnet sich dadurch aus, dass die Wandsegmente wechselweise unter einem Winkel zueinander angeordnete erste Federelemente und zweite Federelemente aufweisen. Hierdurch ergibt sich für die Wandsegmente eine zickzackartige elastische Struktur, wodurch eine gute, elastische Federwirkung erreicht wird.

Die Federelemente sind vorzugsweise in etwa geradlinig ausgebildet. Diese geradlinige Ausbildung ist im Bezug auf eine Projektion auf eine Außenumfangsfläche des Stents zu verstehen.

Die Verbindungselemente verbinden entweder nur erste oder nur zweite Federelemente miteinander, wodurch eine mehr oder weniger gerade Linie gebildet wird. Die Verbindungselemente selbst sind gegenüber entlang der Stentlängsachse einwirkenden Schub- und Zugkräften nicht elastisch und im wesentlichen starr, d. h. dass diese zusammen mit den durch sie verbundenen Federelementen diese Zug- und Schubkräfte aufnehmen und eine Längenänderung des Stents verhindern. Dadurch bilden die jeweils verbundenen ersten bzw. die zweiten Federelemente gemeinsam mit den Verbindungselementen den durchgehenden Längssteg. Zu diesem Zweck sollten die Verbindungselemente und die damit verbundenen Federelemente jeweils parallel zueinander angeordnet sein, d. h. sie liegen innerhalb eines Stents auf einer mehr oder weniger geraden Linie. Weißt der Stent mehr als nur einen Verbindungssteg auf, dann kreuzen bzw. berühren sich die einzelnen Stege nicht, sondern verlaufen parallel in Abstand voneinander. Dies gilt wieder im Bezug auf die Projektion auf eine Umfangsfläche des Stents. Auf diese Weise wird gewährleistet, dass die Krafteinleitung nur entlang des Längsstegs wirkt und keine Querkomponente hat, die zu einer unerwünschten Verkürzung oder Verlängerung des Stents führen könnte.

Eine Ausführungsform der Erfindung zeichnet sich durch mehrere in einer Projektion auf eine Außenumfangsfläche zueinander parallele Längsstege aus, die in Umfangsrichtung voneinander beabstandet angeordnet sind.

Dies können beispielsweise drei oder vier Längsstege sein. Auf diese Weise werden die Wandsegmente besonders wirkungsvoll zueinander positioniert, wobei gleichzeitig eine Längenänderung des Stents zuverlässig vermieden wird.

Es ist auch möglich, dass der Längssteg eine schraubenlinienartige bzw. spiralförmige Gestalt hat und um die Wand des Stents herum achsial verläuft. Dies kann beispielsweise dann der Fall sein, wenn der Längssteg aus den Verbindungselementen und den durch diese verbundenen ersten bzw. zweiten Federelementen besteht. Dies führt zu einem besonders einfachen Aufbau. Längenänderungen aufgrund von Druckspannungen oder Zugspannungen oder durch ein Zusammendrücken des Stents werden trotzdem im Rahmen der beim Gebrauch auftretenden Kraftverhältnisse zuverlässig vermieden.

Gemäß der Erfindung haben die Verbindungselemente insbesonders bezogen auf die Wandflächen auch eine größere Materialdicke bzw. -breite als die Federelemente. Insbesondere dann, wenn ein selbstexpandierender Stent mittels Laser aus einem röhrenförmigen Körper mit geringem Durchmesser geschnitten wird, ergibt sich ein geringer Aufwand. In dem ersten Zustand mit geringem Durchmesser sind die Verbindungselemente dann beispielsweise geringfügig S-förmig ausgebildet. In dem zweiten Zustand mit größerem Durchmesser weisen die Verbindungselemente dann zumindest eine Komponente parallel zu dem Längssteg auf. Die Verbindungselemente können beispielsweise die doppelte Breite der Federelemente haben. Dadurch ergibt sich ein besonders einfaches Schnittmuster. Üblicherweise, jedoch nicht notwendigerweise, sind die Verbindungselemente kürzer als die Federelemente und weisen zweckmäßigerweise eine Länge von max. ¾, vorzugsweise max. 2/3 der Länge des Federelementes auf. Besonders bevorzugt sind Längen von der Hälfte der Federlänge oder weniger.

Vorzugsweise ist der Stent einstückig ausgeführt. Auf diese Weise ergibt sich eine stabile Konstruktion ohne unnötige Kanten oder Sollbruchstellen.

Als Material für den Stent kann ein Formgedächtnis-Material, wie beispielsweise ein sogenanntes Memory-Metall, nämlich eine Nickel-Titan-Legierung verwendet werden, wie sie auch unter dem Namen Nitinol vertrieben wird. Auch Polymere, wie sie in anderen Bereichen der Medizin zur Implantation im Körper verwendet werden, sind zur Herstellung des erfindungsgemäßen Stents geeignet. Aus einem röhrenförmigen Körper mit geringem Durchmesser kann dann beispielsweise mittels Laserstrahlung ein geeignetes Schnittmuster zum Bereitstellen der Federelemente und der Verbindungselemente herausgeschnitten werden. Auf bekannte Weise kann diesem röhrenförmigen Körper dann die expandierte Form aufgeprägt werden. Wird der so erzeugte Stent anschließend in den Zustand mit kleinem Durchmesser komprimiert und beispielsweise mittels eines Katheters in ein erkranktes Blutgefäß eingeführt, so kann der Stent an seiner Position durch Erwärmen über die sog. Konversionstemperatur wieder automatisch in die eingeprägte Form gebracht werden.

Als weitere Materialien für den Stent bieten sich außerdem Edelstahl, Kunststoff oder sog. selbstauflösende Materialien an. Insbesondere diese selbstauflösenden Materialien sind dann von Vorteil, wenn ein Stent nicht dauerhaft gelegt werden soll. Wenn keine selbstexpandierenden Stents verwendet werden, können diese an der gewünschten Position beispielsweise mittels eines Ballonkatheters expandiert werden.

Vorzugsweise sollte die Oberfläche des Stents bearbeitet, insbesondere veredelt, geglättet und/oder poliert sein. Auf diese Weise ergibt sich eine glatte und körperverträgliche Oberfläche. Im folgenden wird ein Ausführungsbeispiel der Erfindung anhand der Zeichnungen näher erläutert. Es zeigen:
- Fig. 1: einen Stent mit den Erfindungsmerkmalen,
- Fig. 2: eine vergrößerte Teilansicht des Stents von Fig.1
- Fig. 3: eine schematische Darstellung eines Schnittmusters zum Herstellen eines Stents mit den Erfindungsmerkmalen,
- Fig. 4: eine schematische Darstellung einer Projektion der Wandsegmente und der Verbindungselemente auf die Außenumfangsfläche der Stents und
- Fig. 5: eine schematische Darstellung ähnlich Fig. 4 eines weiteren Stents mit den Erfindungsmerkmalen.

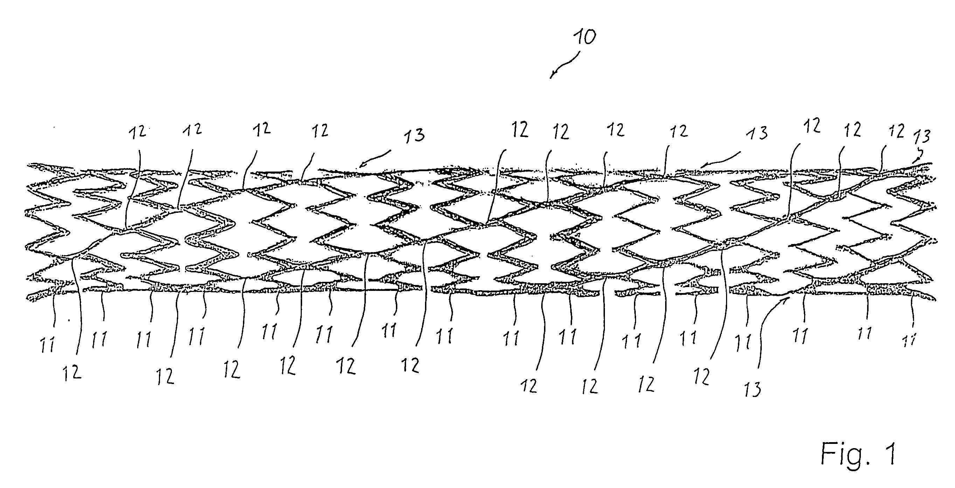

Fig. 1 zeigt eine Draufsicht auf einen Stent 10 mit den Erfindungsmerkmalen gemäß Anspruch 1. Es handelt sich bei dem gezeigten Stent 10 um einen sogenannten flexiblen Stent, der insbesondere in Radialrichtung eine hohe Flexibilität aufweist, um den Bewegungen eines Hohlorganes folgen zu können, in das er eingesetzt ist. Der Stent 10 hat eine insgesamt röhrenförmige Gestalt und weist mehrere Wandsegmente 11 auf, die jeweils eine elastische Struktur haben. Insgesamt haben die Wandsegmente 11 in Fig. 1 jeweils eine zickzackförmige Struktur, die nachfolgend noch näher erläutert wird.

Einander benachbarte Wandsegmente 11 sind mittels Verbindungselementen 12 jeweils miteinander verbunden. Dabei sind die Verbindungselemente 12 derart angeordnet, dass mehrere Verbindungselemente 12 aufeinander folgend einen gemeinsamen durchgehenden Längssteg 13 bilden. In der Fig. 1 sind insgesamt vier durchgehende Längsstege 13 zu erkennen, die sich in etwa schraubenlinienförmig über den Stent 10 erstrekken.

Zugspannungen oder Druckspannungen werden jeweils entlang der Verbindungselemente 12 von einem Wandsegment 11 zum nächsten Wandsegment 11 weitergegeben und so entlang dem durchgehenden Längssteg 13 abgeleitet. Da die Längsstege 13 in Bezug auf eine Projektion auf die Außenumfangsfläche des Stents 10 geradlinig verlaufen, treten bei einer Zugbeanspruchung oder einer Druckbeanspruchung keine Querkomponenten auf. Auf diese Weise führen auftretende Druckspannungen oder Zugspannungen sowie ein Zusammendrücken des Stents 10 nicht zu einer Längenänderung.

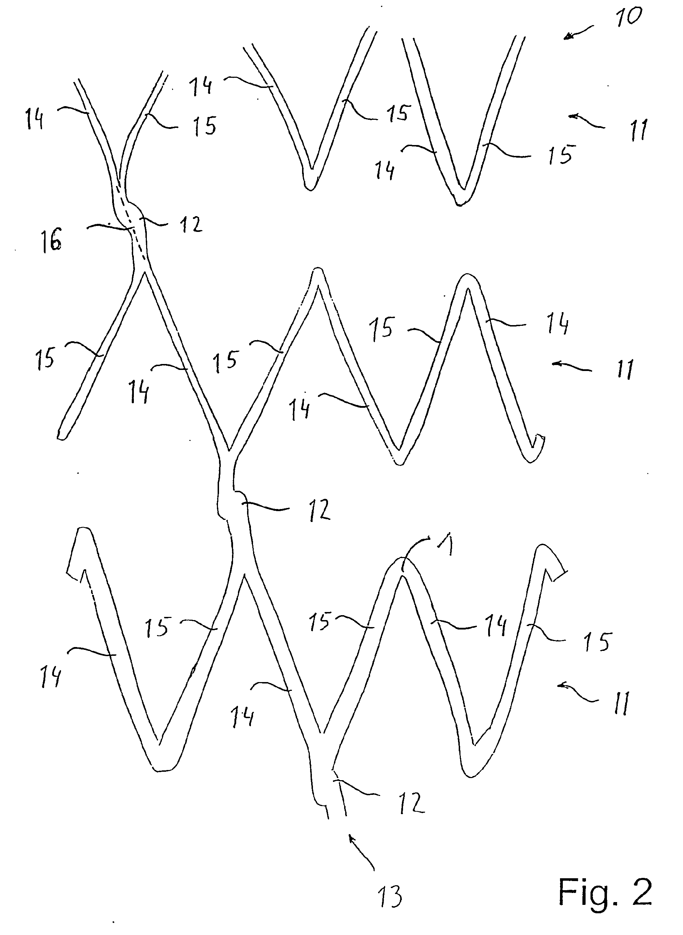

Fig. 2 zeigt eine vergrößerte Teildarstellung des Stents 10 von Fig. 1. Wie der Figur zu entnehmen ist, weisen die Wandsegmente 11 jeweils erste Federelemente 14 und zweite Federelemente 15 auf. Die ersten Federelemente 14 und die zweiten Federelemente 15 sind zueinander jeweils unter einem Winkel angeordnet. Auf diese Weise bilden die ersten Federelemente 14 und die zweiten Federelemente 15 eine zickzackartige Struktur. Dadurch sind die Wandsegmente 11 jeweils in Radialrichtung federnd nachgiebig ausgebildet.

Wie der Fig. 2 außerdem zu entnehmen ist, verbinden die Verbindungselemente 12 jeweils einander benachbarte erste Federelemente 14 der einzelnen Wandsegmente 11 miteinander. Auf diese Weise bilden die Verbindungselemente 12 gemeinsam mit den durch diese verbundenen ersten Federelementen 14 die Längsstege 13. Die einzelnen Wandsegmente 11 sind in der Figur gegen benachbarte Wandsegmente 11 jeweils um einen Versatz versetzt angeordnet, der in etwa der gemeinsamen Dicke des ersten Federelementes 14 und des zweiten Federelementes 15 entspricht. Die Verbindungselemente 12 haben eine in etwa S-förmige Struktur. Auf diese Weise ergibt sich eine in etwa zu den ersten Federelementen 14 parallele Krafteinleitrichtung 16 von einem ersten Federelement 14 zu einem an dieses angrenzende ersten Federelement 14. Die Verbindungselemente 12 weisen außerdem in etwa die doppelte Dicke der ersten Federelemente 14 auf. Wenn in diesem Fall die Krafteinleitung von einem ersten Federelement 14 zum dazu benachbarten ersten Federelement 14 nicht perfekt parallel zu deren Erstreckungsrichtungen erfolgt, lassen sich trotzdem auftretende Querkräfte bis zu einem gewissen Maß durch diese verdickte Ausführung der Verbindungselemente 12 aufnehmen, ohne dass es zu einer Längenänderung des Stents 10 kommt.

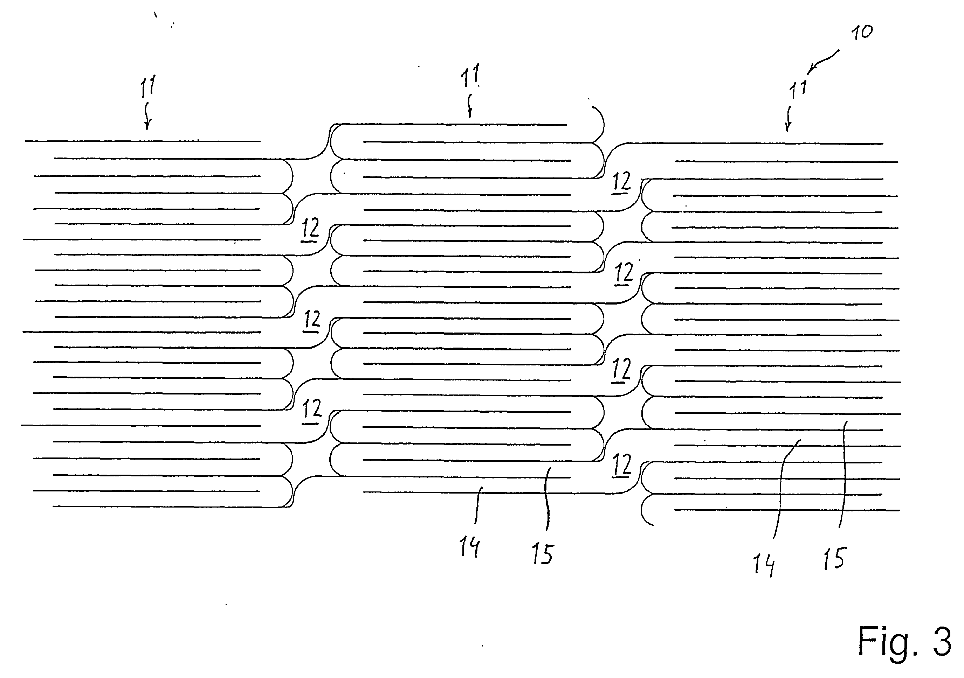

Fig. 3 zeigt eine schematische Darstellung eines Schnittmusters zum Herstellen eines noch nicht expandierten Stents 10. Der Stent 10 wird mittels Laserstrahlung aus einem Röhrchen aus einem geeigneten Material, beispielsweise einem Formgedächtnis-Material, z. B. eines Memory-Metalls, wie Nitinol, ausgeschnitten. Fig. 3 zeigt eine vergrößerte Teildarstellung eines auf die Umfangsfläche des Stents projizierten Schnittmusters in abgewickelter Darstellung. Der leichteren Übersicht halber sind in der Fig. 3 nur zwei erste Federelemente 14 und zwei zweite Federelemente 15 mit Bezugszeichen versehen. Wie sich der Figur entnehmen läßt, sind in einem ersten Zustand nach dem Einschneiden des Schnittmusters in das Röhrchen aus z. B. Memory-Metall die ersten Federelemente 14 und die zweiten Federelemente 15 einander benachbart angeordnet. Die ersten Federelemente 14 und die diesen benachbarten zweiten Federelemente 15 sind dabei parallel aneinander liegend angeordnet. Gut in der Figur zu sehen ist die in etwa S-förmige Gestalt der Verbindungselemente 12. Außerdem wird in der Fig. 3 deutlich, dass einander benachbart angeordnete Wandsegmente 11 jeweils um einen Versatz gegeneinander versetzt angeordnet sind, der der Dicke des ersten Federelementes 14 und des zweiten Federelementes 15 entspricht. Auf diese Weise ist in dem ersten Zustand ein erstes Federelement 14 eines Wandsegmentes 11 an seinen Enden jeweils mittels Verbindungselementen 12 mit einem um den Versatz versetzten ersten Federelements 14 der benachbarten Wandsegmente 11 verbunden. Nach dem Einschneiden des in Fig. 3 dargestellten Schnittmusters in einen röhrenförmigen Rohling aus Memory-Metall wird der so hergestellte Stent in einen zweiten Zustand expandiert, der einen größeren Durchmesser hat als der erste Zustand. Dieser zweite Zustand wird dem Stent 10 dann auf bekannte Weise aufgeprägt. Zur Implantation mittels eines Katheters wird der so vorbereitetet Stent 10 dann in einen Zustand mit geringem Durchmesser zusammengedrückt. Nach der gewünschten Positionierung kann der Stent 10 dann durch Erwärmen über die sogenannten Konversionstemperatur wieder in die eingeprägte Form des zweiten Zustands expandiert werden. Es ist aber auch möglich, den Stent 10 mittels eines Ballonkatheters zu expandieren.

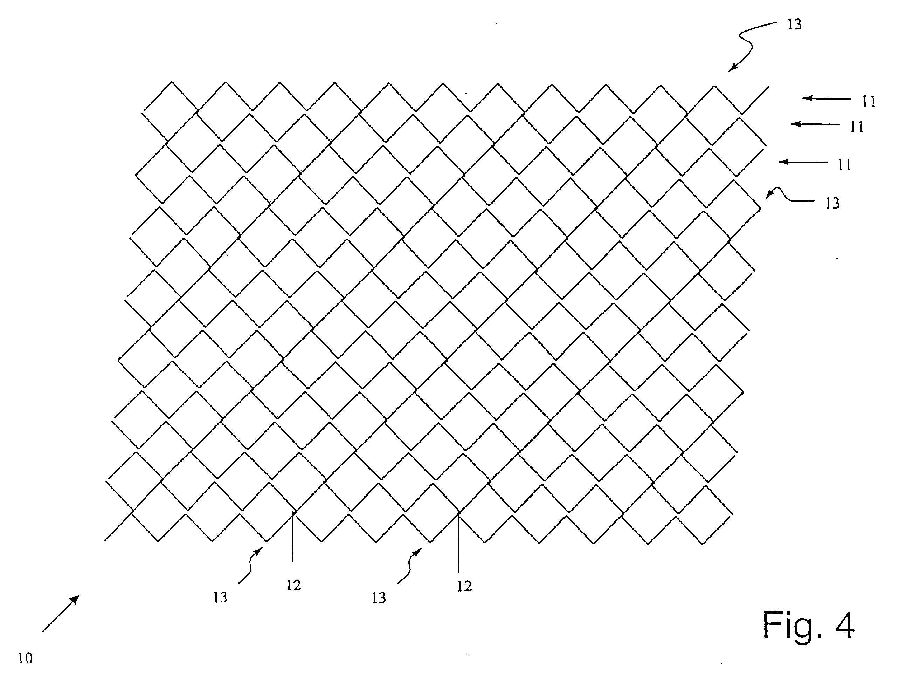

Fig. 4 zeigt eine schematische Darstellung einer Projektion der Wandsegmente 11 und der Verbindungselemente 12 auf die Außenumfangsfläche des Stents 10. Deutlich in der Figur zu sehen ist die jeweils zickzackförmige Ausgestaltung der einzelnen Wandsegmente 11. Der besseren Übersicht halber sind nur drei Wandsegmente 11 in der Figur mit Bezugszeichen versehen. Außerdem deutlich in der Fig. 4 zu erkennen sind die Längsstege 13, die aus abwechselnd aufeinander folgend angeordneten ersten Federelementen 14 und Verbindungselementen 12 ausgebildet sind. Der besseren Übersicht halber sind in der Figur nur zwei Längsstege 13 und zwei Verbindungselemente 12 mit Bezugszeichen versehen. Die Verbindungselemente 12 sind in der Figur als Verdickungen dargestellt. Die Verbindungselemente 12 können beispielsweise auch Punktschweißungen sein.

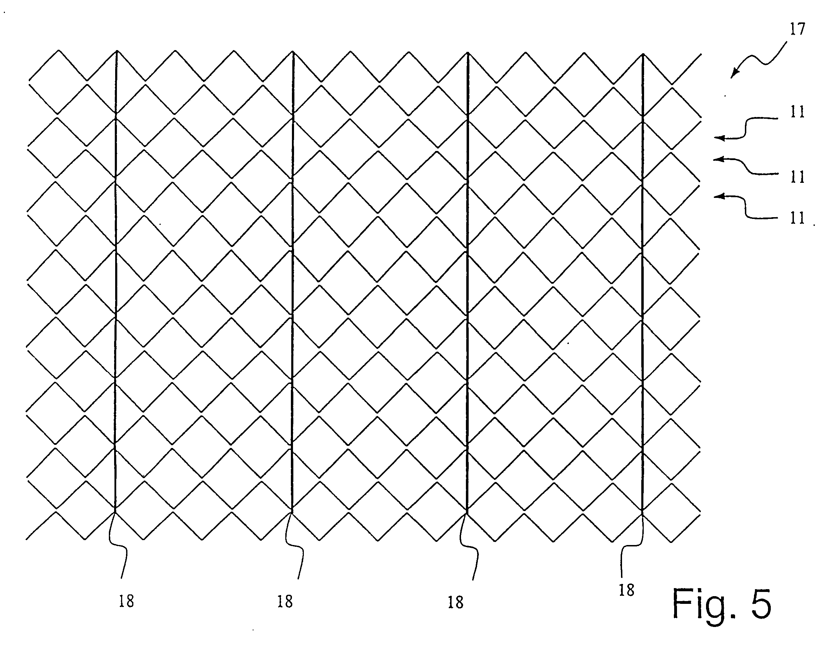

Fig. 5 zeigt eine schematische Darstellung ähnlich Fig. 4 eines weiteren Stents 17 mit den Erfindungsmerkmalen. Gleiche Elemente tragen die gleichen Bezugszeichen wie bei dem Stent 10. Der besseren Übersicht halber sind in der Fig. 5 nur drei Wandsegmente 11 mit Bezugszeichen versehen. Der Stent 17 weist axial verlaufende Längsstege 18 auf. In Fig. 5 sind vier Längsstege 18 dargestellt. Der Stent 17 ist noch widerstandsfähiger gegen Druckspannungen oder Zugspannungen in Längsrichtung als der Stent 10. Allerdings ist die Herstellung des Stents 17 etwas aufwendiger und der Stent 17 ist außerdem etwas weniger flexibel.

### Bezugszeichenliste

- 10: Stent
- 11: Wandsegment
- 12: Verbindungselement
- 13: Längssteg
- 14: erstes Federelement
- 15: zweites Federelement
- 16: Krafteinleitrichtung
- 17: Stent
- 18: Längssteg

## Patentansprüche

1. Stent zur Implantation in oder um ein Hohlorgan, insbesondere selbstexpandierender Stent (10, 17) bei dem unter Zug- und/oder Druckbeanspruchung keine Längenänderung auftritt und der mehrere ringförmige elastische Wandsegmente (11), mit einer radial elastischen Struktur aufweist, wobei die Wandsegmente (11) Federelemente (14, 15) umfassen und mittels Verbindungselementen (12) miteinander verbunden sind, wobei die Verbindungselemente (12) mindestens einen durchgehenden Längssteg (13, 18) bilden, der mindestens eine Komponente in Axialrichtung zum Aufnehmen einer Druckspannung oder einer Zugspannung in Längsrichtung hat, **dadurch gekennzeichnet, dass** die Verbindungselemente (12) eine größere Materialdicke und/oder -breite als die Federelemente (15) aufweisen.

2. Stent nach Anspruch 1, **dadurch gekennzeichnet, dass** die Wandsegmente (11) wechselweise unter einem Winkel zueinander angeordnete erste Federelemente (14) und zweite Federelemente (15) aufweisen.

3. Stent nach Anspruch 2, **dadurch gekennzeichnet, dass** die Federelemente (14, 15) in etwa geradlinig ausgebildet sind.

4. Stent nach Anspruch 2 oder 3, **dadurch gekennzeichnet, dass** die Verbindungselemente (12) entweder nur erste Federelemente (14) oder nur zweite Federelemente (15) miteinander verbinden.

5. Stent nach einem der vorhergehenden Ansprüche, **dadurch gekennzeichnet, dass** mehrere in einer Projektion auf eine Außenumfangsfläche zueinander parallele Längsstege (13, 18) in Umfangsrichtung voneinander beabstandet angeordnet sind.

6. Stent nach einem der vorhergehenden Ansprüche, **dadurch gekennzeichnet, dass** der Längssteg (13) eine schraubenlinienartige Gestalt hat.

7. Stent nach einem der vorherigen Ansprüche, **dadurch gekennzeichnet, dass** die Verbindungselemente (12) die doppelte Breite der Federelemente (14, 15) haben.

8. Stent nach einem der vorhergehenden Ansprüche, **gekennzeichnet durch** eine einstückige Ausführung.

9. Stent nach einem der vorhergehenden Ansprüche, **gekennzeichnet durch** eine Ausführung aus einem Formgedächtnis-Material, insbesondere Nitinol.

10. Stent nach einem der vorhergehenden Ansprüche, **gekennzeichnet durch** eine Ausführung aus Edelstahl, Kunststoff, oder einem selbstauflösenden Material.

11. Stent nach einem der vorhergehenden Ansprüche, **dadurch gekennzeichnet, dass** seine Oberfläche bearbeitet, insbesondere veredelt, geglättet, und/oder poliert ist.

## Claims

1. Stent for implantation in or around a hollow organ, more especially a self-expanding stent (10, 17) where there is no change in length under tensile load and/or compressive load and which includes a plurality of ring-shaped resilient wall segments (11) with a radially resilient structure, wherein the wall segments (11) comprise spring elements (14, 15) and are interconnected by means of connecting elements (12), wherein the connecting elements (12) form at least one continuous longitudinal web (13, 18), which has at least one component for absorbing a compressive stress in the axial direction or a tensile stress in the longitudinal direction, **characterised in that** the material thickness and/or material width of the connecting elements (12) is greater than that of the spring elements (15).

2. Stent according to claim 1, **characterised in that** the wall segments (11) include first spring elements (14) and second spring elements (15) disposed in an alternate manner at an angle one to the other.

3. Stent according to claim 2, **characterised in that** the spring elements (14, 15) are approximately rectilinear.

4. Stent according to claim 2 or 3, **characterised in that** the connecting elements (12) interconnect either only first spring elements (14) or only second spring elements (15).

5. Stent according to one of the preceding claims, **characterised in that** a plurality of longitudinal webs (13, 18) parallel to one another in a projection onto an outside peripheral face are disposed at a spacing from one another in the circumferential direction.

6. Stent according to one of the preceding claims, **characterised in that** the longitudinal web (13) has a helical-type shape.

7. Stent according to one of the preceding claims, **characterised in that** the width of the connecting elements (12) is double the width of the spring elements (14, 15).

8. Stent according to one of the preceding claims, **characterised by** a one-piece embodiment.

9. Stent according to one of the preceding claims, **characterised by** an embodiment produced from a shape memory material, more especially nitinol.

10. Stent according to one of the preceding claims, **characterised by** an embodiment produced from high-grade steel, plastics material or a self-dissolving material.

11. Stent according to one of the preceding claims, **characterised in that** the surface of the said stent is machined, more especially finished, calendered and/or polished.

## Revendications

1. Endoprothèse à implanter dans ou autour d'un organe creux, en particulier une endoprothèse auto-expansible (10, 17) pour laquelle aucune modification de longueur ne se produit sous l'exercice d'une pression et/ou d'une tension et qui comprend plusieurs segments de paroi (11) flexibles circulaires, avec une structure radiale flexible, les segments de paroi (11) comprenant des éléments de ressort (14, 15) et étant reliés les uns aux autres au moyen d'éléments de liaison (12), les éléments de liaison (12) formant au moins une barre longitudinale continue (13, 18), qui possède au moins une composante en direction axiale pour absorber une contrainte de compression ou une contrainte de tension dans le sens de la longueur, **caractérisée en ce que** les éléments de liaison (12) comprennent une largeur et/ou une épaisseur de matériau supérieures à celles des éléments de ressort (15).

2. Endoprothèse selon la revendication 1, **caractérisée en ce que** les segments de paroi (11) comprennent des premiers éléments de ressort (14) et des seconds éléments de ressort (15) disposés alternativement les uns par rapport aux autres suivant un angle.

3. Endoprothèse selon la revendication 2, **caractérisée en ce que** les éléments de ressort (14, 15) sont conçus de manière plus ou moins rectiligne.

4. Endoprothèse selon la revendication 2 ou 3, **caractérisée en ce que** les éléments de liaison (12) relient ensemble soit uniquement les premiers éléments de ressort(14), soit uniquement les seconds éléments de ressort (15).

5. Endoprothèse selon l'une quelconque des revendications précédentes, **caractérisée en ce que** plusieurs barres longitudinales (13, 18) parallèles les unes dans une projection sur une surface périphérique extérieure sont disposées à distance les unes des autres dans la direction périphérique.

6. Endoprothèse selon l'une quelconque des revendications précédentes, **caractérisée en ce que** la barre longitudinale (13) possède une forme hélicoïdale.

7. Endoprothèse selon l'une quelconque des revendications précédentes, **caractérisée en ce que** les éléments de liaison (12) présentent le double de la largeur des éléments de ressort (14, 15).

8. Endoprothèse selon l'une quelconque des revendications précédentes, **caractérisée par** une construction d'un seul tenant.

9. Endoprothèse selon l'une quelconque des revendications précédentes, **caractérisée par** une construction à partir d'un matériau à mémoire de forme, en particulier du Nitinol.

10. Endoprothèse selon l'une quelconque des revendications précédentes, **caractérisée par** une construction à base d'acier inoxydable, de plastique ou d'un matériau s'auto-détruisant.

11. Endoprothèse selon l'une quelconque des revendications précédentes, **caractérisée en ce que** sa surface est traitée, en particulier améliorée, lissée et/ou polie.
